# EUROPÄISCHE PATENTSCHRIFT

(11) **EP 3 413 776 B1**
(45) Veröffentlichungstag und Bekanntmachung des Hinweises auf die Patenterteilung: **25.06.2025**
(21) Anmeldenummer: 17704698.4
(22) Anmeldetag: 07.02.2017
(51) Int. Cl.: A61B 1/00, A61B 1/12, G02B 23/24, G02B 27/00

(54) **VIDEOENDOSKOP**
VIDEO ENDOSCOPE
VIDÉOENDOSCOPE

(30) Priorität: 11.02.2016 DE 102016202093
(43) Veröffentlichungstag der Anmeldung: 19.12.2018
(73) Patentinhaber: Olympus Winter & Ibe GmbH, 22045 Hamburg (DE)
(72) Erfinder: WIETERS, Martin, 22885 Barsbüttel (DE)
(74) Vertreter: Seemann & Partner Patentanwälte mbB
(86) Internationale Anmeldenummer: PCT/EP2017/052579
(87) Internationale Veröffentlichungsnummer: WO 2017/137362

(56) Entgegenhaltungen:
- DE-A1- 102008 031 924
- DE-A1- 102015 101 624
- DE-U1- 202005 019 684
- DE-U1- 202005 019 684
- JP-A- 2014 131 531
- JP-A- H02 257 926
- US-A1- 2010 268 027

## Beschreibung

Die Erfindung betrifft ein Videoendoskop mit einem äußeren Hüllrohr, einem Faserrohr für ein inneres Hüllrohr und mit einem inneren Hüllrohr für eine bildgebende Einheit.

Bei der Montage von Endoskopen werden häufig in ein äußeres Hüllrohr Innenrohre eingeschoben, die im Hinblick auf deren Passung relativ eng ausgelegt sind, um eine Dejustage zu verhindern. Insbesondere bei Videoendoskopen wird beispielsweise die Videoeinheit, die auch als R-Unit bezeichnet wird, in ein äußeres Hüllrohr eingeschoben. Hierbei umfasst die Videoeinheit ein Innenrohr bzw. inneres Hüllrohr, in das optische Elemente und auch eine Videokamera oder zumindest ein lichtsensitiver Chip, wie zum Beispiel ein CCD-Chip, integriert sind. Um eine genaue Justage oder eine genaue Fixierung des Innenrohrs zur optischen Achse zu ermöglichen, ist die Passung zu dem äußeren Hüllrohr bzw. dem Rohr, in das das Innenrohr bzw. das innere Hüllrohr für die bildgebende Einheit eingeschoben wird, relativ eng.

Insbesondere wird bei Videoendoskopen die bildgebende Einheit bzw. die sogenannte R-Unit in das äußere Hüllrohr bzw. in ein Faserrohr geschoben und proximal fixiert. Hierbei ist über das Hüllrohr für die R-Unit ein Schrumpfschlauch gezogen, wobei der Schrumpfschlauch dazu dient, ein Klappern des inneren Hüllrohrs zu verhindern und darüber hinaus auch eine elektrische Isolation zu bewirken. Auf das Faserrohr für das innere Hüllrohr ist eine Heizfolie umlaufend über den Umfang des Faserrohrs aufgebracht.

In DE 20 2005 019 684 U1 ist eine endoskopartige Vorrichtung offenbart, bei der in einer Röhre ein Heizungssystem integriert ist, mit welchem dem Mündungsbereich der Röhre Wärme zugeführt wird. Des Weiteren ist in die Röhre ein elektrisches Heizelement eingebracht.

Ferner offenbart US 2010/0268027 A1 ein Endoskop, wobei am distalen Ende des Endoskops eine Heizeinheit innerhalb eines Halteelements vorgesehen ist. Außerdem umfasst die Heizeinheit ein Heizelement, das an einem distalen Sichtfenster angeordnet ist.

Des Weiteren beschreibt DE 10 2008 031 924 A1 ein Endoskop mit einer Widerstandsheizung, wobei die Heizung auf der Außenfläche des Faserrohrs als Heizfolie ausgebildet ist.

Das nachveröffentlichte Dokument DE 10 2015 101 624 A1 offenbart ein Videoendoskop, wobei im Bereich zwischen einer unteren Kante einer Prismenbaugruppe und einer Wand eines Faserrohrs ein Hohlraum vorgesehen ist. In diesem Hohlraum kann beispielsweise ein Heizelement angeordnet werden.

Zudem ist in JP 2014-131531 A ist ein Anti-Beschlagsystem für ein Endoskop mit einer Heizung beschrieben.

Ausgehend von diesem Stand der Technik besteht die Aufgabe der Erfindung darin, eine kostengünstige Montage von Videoendoskopen zu ermöglichen, wobei es möglich sein soll, gegebenenfalls mehr Bauraum für andere Bestandteile des Endoskops bereitzustellen.

Gelöst wird diese Aufgabe durch ein Videoendoskop mit einem äußeren Hüllrohr, wobei in dem äußeren Hüllrohr ein Faserrohr für ein inneres Hüllrohr aufgenommen ist, wobei in dem Faserrohr ein inneres Hüllrohr für eine bildgebende Einheit aufgenommen ist und wobei zwischen dem Faserrohr und dem inneren Hüllrohr eine das innere Hüllrohr umschließende Heizfolie vorgesehen ist.

Das erfindungsgemäße Videoendoskop weist ein äußeres Hüllrohr und ein inneres Hüllrohr für eine bildgebende Einheit auf. Ferner ist ein Faserrohr für das innere Hüllrohr vorgesehen, wobei in dem äußeren Hüllrohr das Faserrohr aufgenommen ist und in dem Faserrohr das innere Hüllrohr aufgenommen ist. Außerdem ist zwischen dem das innere Hüllrohr aufnehmenden Faserrohr und dem inneren Hüllrohr eine Heizfolie, die das innere Hüllrohr umschließt, angeordnet.

Die Erfindung beruht auf dem Gedanken, dass bei dem erfindungsgemäßen Videoendoskop eine Heizfolie anstelle des bisherigen Schrumpfschlauches auf das innere Hüllrohr für die bildgebende Einheit bzw. die R-Unit angebracht wird, so dass die Heizfolie zwischen dem inneren Hüllrohr und dem das innere Hüllrohr umschließenden Faserrohr angebracht wird oder ist, so dass die Heizfolie nicht nur zum Heizen eingesetzt wird, sondern darüber hinaus auch als Klapperschutz für das innere Hüllrohr ausgebildet ist. Ferner kann die Heizfolie auch als elektrische Isolation eingesetzt werden. Dies hängt von der Geometrie des Bauraums im Endoskopschaft bzw. im äußeren Hüllrohr ab.

Dadurch, dass die Heizfolie zwischen dem Faserrohr und dem inneren Hüllrohr (anstelle des bisherigen Schrumpfschlauches) angeordnet wird, wird auf den Schrumpfschlauch bei der Montage des Videoendoskops verzichtet, wodurch ein Bauteil (Schrumpfschlauch) entfällt. Außerdem wird dadurch die Montage des Endoskops vereinfacht, da ein Montageschritt entfällt. Überdies wird durch das Wegfallen des Schrumpfschlauches eine Durchmesserreduzierung erreicht, so dass hierdurch mehr Bauraum zum Beispiel für Lichtleitfasern oder die Optik entsteht oder stabilere Rohre verwendet werden können. Außerdem ist es möglich, aufgrund der Durchmesserreduzierung Videoendoskope bereitzustellen, die einen geringeren Durchmesser des Endoskopschafts aufweisen.

Durch die Heizfolie wird beispielsweise ein distales Fenster eines Endoskops beheizt, um ein Beschlagen des Fensters zu vermeiden. Hierbei wird mittels der Heizfolie eine elektrische Widerstandsheizung bereitgestellt, die vorzugsweise in der Nähe des Fensters im Endoskopschaft angeordnet ist. Durch den Kontakt der Heizfolie mit dem Faserrohr, das üblicherweise aus Metall besteht oder hergestellt ist, wird ein guter Wärmetransport durch das Faserrohr zum Fenster, das beispielsweise im Faserrohr vorzugsweise in gutem Wärmekontakt eingelötet ist, erreicht.

Dazu ist in einer Weiterbildung vorgesehen, dass das innere Hüllrohr einen Rohrabschnitt aufweist, wobei der Rohrabschnitt des inneren Hüllrohrs von der Heizfolie in Umfangsrichtung umgeben ist. Dadurch umschließt die Heizfolie den Rohrabschnitt des inneren Hüllrohrs.

Des Weiteren zeichnet sich das Videoendoskop in einer Weiterbildung dadurch aus, dass die Heizfolie schlauchförmig oder als Heizfolienschlauch ausgebildet ist.

Vorzugsweise ist die Heizfolie in unmittelbarem oder direktem Kontakt mit dem Faserrohr und mit dem inneren Hüllrohr.

Insbesondere ist die Heizfolie aus Kunststoff, insbesondere Polyimid, hergestellt. Hierbei eigenen sich insbesondere Heizfolien aus Kapton^{®}.

Außerdem ist in einer Ausgestaltung des Videoendoskops weiter vorgesehen, dass im äußeren Hüllrohr neben dem Faserrohr für das innere Hüllrohr ein Lichtleitfaserbündel aufgenommen ist.

Vorzugsweise ist bei dem Videoendoskop in einer Weiterbildung wenigstens ein Temperatursensor zur Erfassung der Temperatur der Heizfolie vorgesehen, wobei insbesondere die Heizfolie den wenigstens einen Temperatursensor aufweist. Mittels des Temperatursensors wird die Temperatur der Heizfolie in dem Endoskopschaft erfasst oder ermittelt, so dass es ermöglicht wird, in Abhängigkeit der erfassten Heizfolientemperatur eine vorbestimmte Temperatur mittels einer Regeleinrichtung oder eines Regelkreises einzustellen.

Dazu ist in einer Ausführungsform des Videoendoskops vorgesehen, dass eine Temperaturregeleinrichtung zur Regelung der Temperatur der Heizfolie vorgesehen ist. Hierbei ist die Temperaturregeleinrichtung vorzugsweise mit einem Temperatursensor der Heizfolie oder für die Heizfolie verbunden, um von diesem Temperatursensor eine gemessene Ist-Temperatur zu empfangen, so dass mittels eines Ist-Sollwert-Vergleichs in der mit der Heizfolie verbundenen Temperaturregeleinrichtung eine vorbestimmte Temperatur der Heizfolie eingestellt oder eingeregelt wird.

Vorzugsweise weist die Heizfolie einen positiven Temperaturkoeffizienten des elektrischen Widerstands auf.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: schematisch einen Querschnitt durch einen distalen Endbereich eines Endoskopschafts gemäß dem Stand der Technik und
- Fig. 2: schematisch einen Querschnitt durch einen distalen Endbereich eines erfindungsgemäßen Endoskopschafts.
- Fig. 3: schematisch einen Querschnitt durch einen distalen Endbereich eines erfindungsgemäßen Endoskopschafts mit einer Temperaturregelung.

In den Zeichnungen sind jeweils gleiche oder gleichartige Elemente und/oder Teile mit denselben Bezugsziffern versehen, so dass von einer erneuten Vorstellung jeweils abgesehen wird.

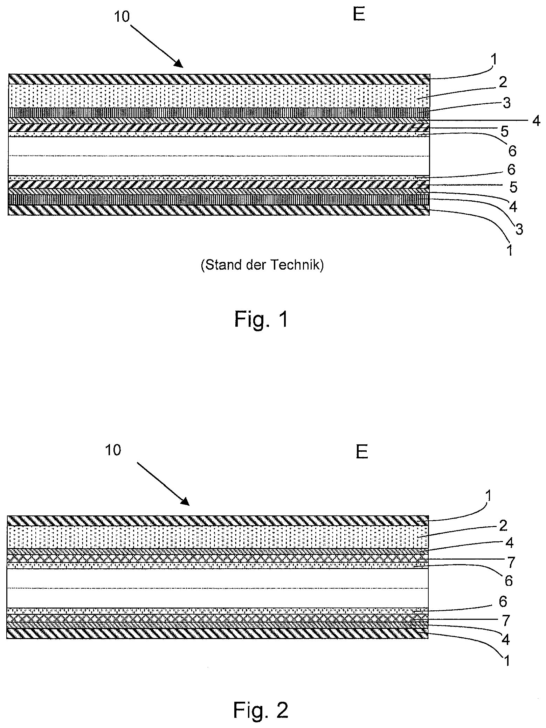

Fig. 1 zeigt schematisch einen Querschnitt durch einen Endoskopschaft 10 eines schematisch bezeichneten Videoendoskops E im Ausschnitt, wobei in Fig. 1 der Ausschnitt einen distalen Endbereich des Endoskopschafts 10 zeigt.

Der Endoskopschaft 10 weist ein äußeres Hüllrohr 1 auf, das im Inneren ein von einer Heizfolie 3 umhülltes Faserrohr 4 umgibt. Das Faserrohr 4 ist hierbei exzentrisch im äußeren Hüllrohr 1 angeordnet. Zwischen dem Faserrohr 4 bzw. der äußeren Heizfolie 3 und dem äußeren Hüllrohr 1 ist der Zwischenraum mit Lichtleitfasern 2 gefüllt, die distal in der Stirnfläche des Videoendoskops E enden.

Das Faserrohr 4 umschließt ein im Inneren des Faserrohrs 4 angeordnetes inneres Hüllrohr 6 für eine bildgebende Einheit (hier nicht dargestellt) für das Videoendoskop E. Hierbei kann die bildgebende Einheit als sogenannte R-Unit zum Beispiel mit einem CCD-Chip ausgebildet sein.

Zwischen dem Faserrohr 4 und dem inneren Hüllrohr 6 ist ein Schrumpfschlauch 5 angeordnet, der das innere Hüllrohr 6 umschließt.

Um ein Beschlagen eines distalen Fensters (hier nicht dargestellt) des Videoendoskops E zu verhindern, ist das Faserrohr 4 von der Heizfolie 3 umgeben, die um die Außenfläche des Faserrohrs 4 gewickelt ist.

Die Heizfolie 3 ist dabei über ein (hier nicht dargestelltes) Kabel angeschlossen, um einen Heizwiderstand in der Heizfolie 3 mit Strom zu beaufschlagen, wodurch die Heizfolie 3 und das von der Heizfolie 3 umgebene Faserrohr 4 geheizt wird, so dass ein stirnseitiges Fenster des Videoendoskops E geheizt wird.

In Fig. 2 ist schematisch ein Abschnitt eines erfindungsgemäßen Endoskopschafts 10 des Videoendoskops E im Querschnitt dargestellt. Hierbei ist der distale Endbereich des Endoskopschafts 10 in Fig. 2 schematisch gezeigt. Im Inneren des äußeren Hüllrohrs 1 ist ein Faserrohr 4 angeordnet, wobei im Inneren des Faserrohrs 4 das langgestreckte innere Hüllrohr 6 angeordnet ist. Zwischen dem Faserrohr 4 und dem inneren Hüllrohr 6 ist eine Heizfolie 3 angeordnet, die in direktem Kontakt mit dem Faserrohr 4 und dem inneren Hüllrohr 6 ist. Vorzugsweise ist die Heizfolie 7 aus Kapton^{®} ausgebildet. Darüber hinaus sind auch entsprechende Anschlussmittel für die Widerstandsheizung der Heizfolie 7 vorgesehen, um eine Erwärmung des Faserrohrs 4 zu bewirken.

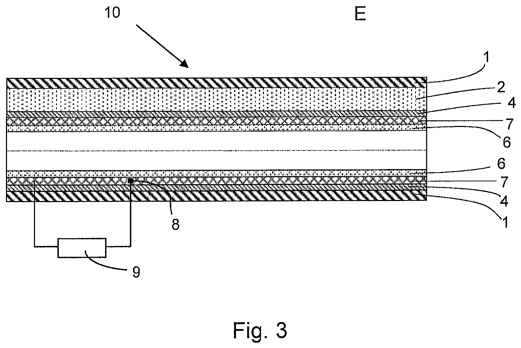

In Fig. 3 ist schematisch ein Querschnitt durch einen Endoskopschaft dargestellt, wobei gegenüber dem Ausführungsbeispiel in Fig. 2 für die Heizfolie 7 eine Temperaturregelung möglich ist. Die Heizfolie 7 weist einen Temperatursensor 8 auf, mittels dem die Temperatur der Heizfolie 7 erfasst wird. Bei Bestromung der Heizfolie 7 wird diese erwärmt, so dass die von dem Temperatursensor 8 ermittelte Temperatur der Heizfolie 7 an eine Temperaturregeleinrichtung 9 übermittelt wird. Nach einem Ist-Sollwert-Vergleich in der Temperaturregeleinrichtung 9 wird die Stärke des elektrischen Stroms für die Heizfolie 7 entsprechend geregelt, so dass eine bevorzugte Temperatur in der Heizfolie eingestellt und konstant gehalten wird.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 1: äußeres Hüllrohr
- 2: Lichtleitfasern
- 3: Heizfolie
- 4: Faserrohr
- 5: Schrumpfschlauch
- 6: inneres Hüllrohr
- 7: Heizfolie
- 8: Temperatursensor
- 9: Temperaturregeleinrichtung
- 10: Endoskopschaft

- E: Videoendoskop

## Patentansprüche

1. Videoendoskop (E) mit einem äußeren Hüllrohr (1), wobei in dem äußeren Hüllrohr (1) ein Faserrohr (4) für ein inneres Hüllrohr (6) aufgenommen ist, wobei in dem Faserrohr (4) ein inneres Hüllrohr (6) für eine bildgebende Einheit aufgenommen ist und wobei zwischen dem Faserrohr (4) und dem inneren Hüllrohr (6) eine das innere Hüllrohr (6) umschließende Heizfolie (7) vorgesehen ist.

2. Videoendoskop (E) nach Anspruch 1, **dadurch gekennzeichnet, dass** das innere Hüllrohr (6) einen Rohrabschnitt aufweist, wobei der Rohrabschnitt des inneren Hüllrohrs (6) von der Heizfolie (7) in Umfangsrichtung umgeben ist.

3. Videoendoskop (E) nach Anspruch 1 oder 2, **dadurch gekennzeichnet, dass** die Heizfolie (7) schlauchförmig oder als Heizfolienschlauch ausgebildet ist.

4. Videoendoskop (E) nach einem der Ansprüche 1 bis 3, **dadurch gekennzeichnet, dass** die Heizfolie (7) in unmittelbarem oder direktem Kontakt mit dem Faserrohr (4) und mit dem inneren Hüllrohr (6) ist.

5. Videoendoskop (E) nach einem der Ansprüche 1 bis 4, **dadurch gekennzeichnet, dass** die Heizfolie (7) aus Kunststoff, insbesondere aus Polyimid hergestellt ist.

6. Videoendoskop (E) nach einem der Ansprüche 1 bis 5, **dadurch gekennzeichnet, dass** im äußeren Hüllrohr (1) neben dem Faserrohr (4) für das innere Hüllrohr (6) ein Lichtleitfaserbündel (2) aufgenommen ist.

7. Videoendoskop (E) nach einem der Ansprüche 1 bis 6, **dadurch gekennzeichnet, dass** wenigstens ein Temperatursensor (8) zur Erfassung der Temperatur der Heizfolie (7) vorgesehen ist, wobei insbesondere die Heizfolie (7) den wenigstens einen Temperatursensor (7) aufweist.

8. Videoendoskop (E) nach einem der Ansprüche 1 bis 7, **dadurch gekennzeichnet, dass** eine Temperaturregeleinrichtung (9) zur Regelung der Temperatur der Heizfolie (7) vorgesehen ist.

9. Videoendoskop (E) nach einem der Ansprüche 1 bis 8, **dadurch gekennzeichnet, dass** die Heizfolie (7) einen positiven Temperaturkoeffizienten des elektrischen Widerstands aufweist.

## Claims

1. A video endoscope (E) with an outer casing tube (1), wherein a fiber tube (4) for an inner casing tube (6) is accommodated in the outer casing tube (1), wherein an inner casing tube (6) for an image-forming unit is accommodated in the fiber tube (4) and wherein a heating film (7) surrounding the inner casing tube (6) is provided between the fiber tube (4) and the inner casing tube (6).

2. The video endoscope (E) according to claim 1, **characterized in that** the inner casing tube (6) has a tube section, wherein the tube section of the inner casing tube (6) is enclosed by the heating film (7) in the circumferential direction.

3. The video endoscope (E) according to claim 1 or 2, **characterized in that** the heating film (7) is designed sleeve-shaped or as a heating film sleeve.

4. The video endoscope (E) according to one of claims 1 to 3, **characterized in that** the heating film (7) is in immediate or direct contact with the fiber tube (4) and with the inner casing tube (6).

5. The video endoscope (E) according to one of claims 1 to 4, **characterized in that** the heating film (7) is produced from plastic, in particular from polyimide.

6. The video endoscope (E) according to one of claims 1 to 5, **characterized in that** an optical fiber bundle (2) is accommodated in the outer casing tube (1) next to the fiber tube (4) for the inner casing tube (6).

7. The video endoscope (E) according to one of claims 1 to 6, **characterized in that** at least one temperature sensor (8) for detecting the temperature of the heating film (7) is provided, wherein in particular the heating film (7) has the at least one temperature sensor (8).

8. The video endoscope (E) according to one of claims 1 to 7, characterized that a temperature control device (9) for controlling the temperature of the heating film (7) is provided.

9. The video endoscope (E) according to one of claims 1 to 8, **characterized in that** the heating film (7) has a positive temperature coefficient of the electrical resistance.

## Revendications

1. Vidéo-endoscope (E) comprenant un tube enveloppe extérieur (1), un tube en fibre (4) étant reçu dans le tube enveloppe extérieur (1) pour former un tube enveloppe intérieur (6), un tube enveloppe intérieur (6) pour une unité d'imagerie étant reçu dans le tube en fibre (4), et une feuille chauffante (7) entourant le tube enveloppe intérieur (6) étant prévue entre le tube en fibre (4) et le tube enveloppe intérieur (6).

2. Vidéo-endoscope (E) selon la revendication 1, **caractérisé en ce que** le tube enveloppe intérieur (6) présente un segment de tube, le segment de tube du tube enveloppe intérieur (6) étant entouré, sur sa périphérie, par la feuille chauffante (7).

3. Vidéo-endoscope (E) selon la revendication 1 ou la revendication 2, **caractérisé en ce que** la feuille chauffante (7) est conçue en forme de tuyau ou en tant que tuyau à feuille chauffante.

4. Vidéo-endoscope (E) selon l'une des revendications 1 à 3, **caractérisé en ce que** la feuille chauffante (7) est en contact direct ou immédiat avec le tube en fibre (4) et avec le tube enveloppe intérieur (6).

5. Vidéo-endoscope (E) selon l'une des revendications 1 à 4, **caractérisé en ce que** la feuille chauffante (7) est fabriquée en matière plastique, en particulier en polyimide.

6. Vidéo-endoscope (E) selon l'une des revendications 1 à 5, **caractérisé en ce qu'**un faisceau de fibres optiques (2) est logé dans le tube enveloppe extérieur (1), à côté du tube en fibre (4) pour le tube enveloppe intérieur (6).

7. Vidéo-endoscope (E) selon l'une des revendications 1 à 6, **caractérisé en ce qu'**au moins un capteur de température (8) est prévu pour détecter la température de la feuille chauffante (7), la feuille chauffante (7) comportant notamment ledit au moins un capteur de température (7).

8. Vidéo-endoscope (E) selon l'une des revendications 1 à 7, **caractérisé en ce qu'**un dispositif de régulation de température (9) est prévu pour réguler la température de la feuille chauffante (7).

9. Vidéo-endoscope (E) selon l'une des revendications 1 à 8, **caractérisé en ce que** la feuille chauffante (7) présente un coefficient de température positif de la résistance électrique.
